# EUROPEAN PATENT APPLICATION

(11) **EP 4 382 166 A1**
(43) Date of publication of application: **12.06.2024**
(21) Application number: 22852325.4
(22) Date of filing: 05.08.2022
(51) Int. Cl.: A61P 35/00, A61K 39/395, C07K 16/28, C12N 15/13, A61K 47/68, C12N 15/62, C07K 19/00

(54) **ANTI-PD-L1 NANOBODY AND USE THEREOF**

(30) Priority: 06.08.2021 CN 202110903293; 30.09.2021 CN 202111162303
(71) Applicant: Betta Pharmaceuticals Co., Ltd, Yuhang Hangzhou Zhejiang 311100 (CN)
(72) Inventor: XU, Wenxin, Hangzhou, Zhejiang 311100 (CN); FANG, Xuefei, Hangzhou, Zhejiang 311100 (CN); XU, Deyu, Hangzhou, Zhejiang 311100 (CN); DING, Lieming, Hangzhou, Zhejiang 311100 (CN)
(74) Representative: J A Kemp LLP
(86) International application number: PCT/CN2022/110423
(87) International publication number: WO 2023/011614

(57) **Abstract**

Provided are an anti-PD-L1 nanobody and use thereof. The anti-PD-L1 nanobody comprises a CDR1, a CDR2, and a CDR3, wherein the CDR1 comprises a sequence selected from SEQ ID NOs: 1, 5, and 9; the CDR2 comprises a sequence selected from SEQ ID NOs: 2, 6, and 10; and the CDR3 comprises a sequence selected from SEQ ID NOs: 3, 7, 11, and 14. The antibody can block the PD-1/PD-L1 and CD-80/PD-L1 signaling pathways, inhibit the growth of tumor cells, and exhibit a therapeutic effect against tumors.

## Description

### CROSS-REFERENCE TO RELATED APPLICATIONS

The present invention claims priority to Chinese Patent Application No. 202110903293.4 filed on August 6, 2021 and Chinese Patent Application No. 202111162303.X filed on September 30, 2021, which are incorporated herein by reference in their entirety or in part.

### TECHNICAL FIELD

The present invention relates to the field of biopharmaceutics and in particular to an anti-PD-L1 nanobody and use thereof.

### BACKGROUND

Programmed death ligand 1 (PD-L1) plays a key role as a negative regulator of anti-tumor immunity. It can bind to programmed death-1 (PD-1), induce tumor-specific T cell apoptosis by inhibiting T cell activation, and play roles such as immune escape, immunosuppression, etc. in pathological conditions such as tumors, chronic inflammation, etc. PD-L1 can restore and enhance T cell function by blocking the interaction of PD-1/PD-L1, thereby playing a role in tumor treatment and immunoregulation. Meanwhile, the co-inhibition function of CD80 and PD-L1 can also be blocked to facilitate the full activation of T cell function and cytokine production. As research has advanced, various monoclonal antibodies against PD-L1, such as atezolizumab (under the trade name Tecentriq), durvalumab, avelumab (under the trade name Bavencio), etc., have been applied to the treatment of various types of solid tumors, such as non-small cell lung cancer, melanoma, lymphoma, head and neck cancer, stomach cancer, etc., and have shown good therapeutic effects against tumors.

Using monoclonal antibodies as immune checkpoint inhibitors has become an effective means of treating various types of cancer. However, as conventional monoclonal antibodies are generally large in molecular weight (approximately 150 kDa), their tissue permeability is relatively poor after administration, and their immunogenicity is relatively high, which limits their function of targeting tumors of interest. Nanobodies are heavy chain-only antigen-binding fragments derived from the family Camelidae, and they are capable of functioning without light chains. Nanobodies are small in molecular weight (typically 12-16 KDa) and have also retained the binding activity to antigens; moreover, they do not need intrachain disulfide bonds. Nanobodies have gradually emerged as a key player in immunotherapy. Nanobodies can be readily expressed in many different expression systems, e.g., prokaryotic expression systems (such as Escherichia coli), eukaryotic expression systems (such as yeast), mammalian cell expression systems (such as CHO and HEK-293 cells), etc., and can be readily purified. In September 2018, the world's first nanobody caplacizumab was approved in the European Union as a treatment for acquired thrombotic thrombocytopenic purpura (aTTP) in adult patients. Nanobodies have shown unique development and application value in various aspects such as cancer, autoimmune diseases, respiratory diseases, hematologic diseases, etc., and more and more pharmaceutical enterprises are paying attention to the development of nanobodies.

However, the development of nanobodies is not as common as that of conventional monoclonal antibodies. There remains difficulty in screening for nanobodies with high affinity, high specificity, and prospects for industrialization, which limits the research and development of nanobodies to some extent.

### SUMMARY

The present invention provides an anti-PD-L1 nanobody and use thereof in treating disease. The present invention provides an anti-PD-L1 nanobody, a fusion protein comprising the PD-L1 nanobody, and use of the nanobody or the fusion protein in treating diseases, particularly cancers.

A first aspect of the present invention provides an anti-PD-L1 nanobody, comprising a CDR1, a CDR2, and a CDR3, wherein (a) the CDR1 comprises a sequence selected from the group consisting of SEQ ID NOs: 1, 5, and 9, or sequences that have one or two amino acid substitutions, deletions, or additions compared to SEQ ID NOs: 1, 5, and 9; (b) the CDR2 comprises a sequence selected from the group consisting of: SEQ ID NOs: 2, 6, and 10, or sequences that have one or two amino acid substitutions, deletions, or additions compared to SEQ ID NOs: 2, 6, and 10; (c) the CDR3 comprises a sequence selected from the group consisting of: SEQ ID NOs: 3, 7, 11, and 14, or sequences that have one, two, or three amino acid substitutions, deletions, or additions compared to SEQ ID NOs: 3, 7, 11, and 14.

The present invention further provides an anti-PD-L1 nanobody, selected from: i) an antibody that has the amino acid sequence set forth in SEQ ID NO: 4, or SEQ ID NO: 8, or SEQ ID NO: 12, or SEQ ID NO: 13, or SEQ ID NO: 15, or SEQ ID NO: 28, or SEQ ID NO: 29, or SEQ ID NO: 30, or SEQ ID NO: 31, or SEQ ID NO: 32; or ii) an antibody that has an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology compared to i).

According to the examples of the present invention, the anti-PD-L1 nanobody provided comprises: (1) a CDR1, which is the amino acid sequence set forth in SEQ ID NO: 1; a CDR2, which is the amino acid sequence set forth in SEQ ID NO: 2; and a CDR3, which is the amino acid sequence set forth in SEQ ID NO: 3; or (2) a CDR1, which is the amino acid sequence set forth in SEQ ID NO: 5; a CDR2, which is the amino acid sequence set forth in SEQ ID NO: 6; and a CDR3, which is the amino acid sequence set forth in SEQ ID NO: 7; or (3) a CDR1, which is the amino acid sequence set forth in SEQ ID NO: 9; a CDR2, which is the amino acid sequence set forth in SEQ ID NO: 10; and a CDR3, which is the amino acid sequence set forth in SEQ ID NO: 11; or (4) a CDR1, which is the amino acid sequence set forth in SEQ ID NO: 1; a CDR2, which is the amino acid sequence set forth in SEQ ID NO: 2; and a CDR3, which is the amino acid sequence set forth in SEQ ID NO: 14.

A second aspect of the present invention provides a fusion protein, wherein the fusion protein comprises: a first domain, which is the anti-PD-L1 nanobody according to the first aspect of the present invention; and a second domain, which is a functional protein different from the anti-PD-L1 nanobody.

A third aspect of the present invention provides an antibody conjugate, wherein the antibody conjugate comprises the anti-PD-L1 nanobody according to the first aspect of the present invention or the fusion protein according to the second aspect of the present invention; and a functional small molecule linked to the anti-PD-L1 nanobody or the fusion protein.

A fourth aspect of the present invention provides an isolated polynucleotide, wherein the isolated polynucleotide encodes the anti-PD-L1 nanobody according to the first aspect or encodes the fusion protein according to the second aspect.

A fifth aspect of the present invention provides a construct, wherein the construct comprises the isolated polynucleotide according to the fourth aspect described above.

A sixth aspect of the present invention provides a host cell, wherein the host cell comprises the isolated polynucleotide according to the fourth aspect described above or the construct according to the fifth aspect described above.

A seventh aspect of the present invention provides a method for producing a nanobody or a fusion protein, comprising: culturing a host cell, wherein the host cell is the host cell according to the sixth aspect of the present invention; and collecting from the culture the nanobody or the fusion protein.

An eighth aspect of the present invention provides a pharmaceutical composition, comprising the anti-PD-L1 nanobody according to the first aspect described above, or the fusion protein according to the second aspect described above, or the antibody conjugate according to the third aspect described above; and a pharmaceutically acceptable carrier.

A ninth aspect of the present invention provides a kit, comprising the anti-PD-L1 nanobody according to the first aspect described above, or the fusion protein according to the second aspect described above, or the antibody conjugate according to the third aspect described above.

A tenth aspect of the present invention provides a method for preventing and/or treating disease, comprising: administering to a subject a prophylactically and/or therapeutically effective amount of the anti-PD-L1 nanobody according to the first aspect described above, or the fusion protein according to the second aspect, or the antibody conjugate according to the third aspect.

An eleventh aspect of the present invention provides use of an anti-PD-L1 nanobody or a fusion protein or an antibody conjugate in preparing a medicament or a kit, wherein the anti-PD-L1 nanobody is the anti-PD-L1 nanobody according to the first aspect of the present invention; the fusion protein is the fusion protein according to the second aspect of the present invention; the antibody conjugate is the antibody conjugate according to the third aspect of the present invention.

### BRIEF DESCRIPTION OF THE DRAWINGS

FIG. 1 shows the results of the FACS assay for the binding activity of the antibodies to hPD-L1, provided in Example 2 of the present invention.
FIG. 2 shows the results of the ELISA assay for the binding activity of the antibodies to cynomolgus monkey PD-L1, provided in Example 2 of the present invention.
FIG. 3 shows the results of the FACS assay for the blocking activity of the antibodies against PD1/PD-L1, provided in Example 3 of the present invention.
FIG. 4 shows the results of the FACS assay for the blocking activity of the antibodies against CD80/PD-L1, provided in Example 3 of the present invention.
FIG. 5 shows the results of the reporter gene assay for the blocking of the PD-L1/PD-1 pathway by the antibodies, provided in Example 4 of the present invention.
FIG. 6 shows the efficacy results of the antibodies in mice, provided in Example 7 of the present invention.

### DETAILED DESCRIPTION

Certain terms used herein are explained and described below. These explanations and descriptions are merely used to facilitate understanding by those skilled in the art and should not be construed as limiting the protection scope of the present invention.

The term "antibody" is used herein in its broadest sense to refer to a protein or polypeptide that comprises an antigen-binding site or antigen-binding fragment or antigen-binding moiety, and to encompass natural and artificial antibodies of various structures, including but not limited to intact antibody forms or antigen-binding fragments of antibodies. According to a specific embodiment, the antibody provided is a nanobody.

The nanobody mentioned herein has the same meaning as a single-domain antibody and refers to an antibody consisting of only one heavy chain variable region, which is the smallest antigen-binding fragment with full function. The nanobody comprises the four conserved framework regions (FR1-FR4) and three complementarity-determining regions (CDR1-CDR3) mentioned below. The framework region FR1, the framework region FR2, the framework region FR3, and the framework region FR4 are separated by the complementarity-determining region CDR1, the complementarity-determining region CDR2, and the complementarity-determining region CDR3. The complementarity-determining regions are also referred to as "hypervariable regions". The complementarity-determining regions determine the characteristics of binding to the antigen.

"Humanized antibody" refers to antibodies consisting of molecules based on antigen-binding sites derived from non-human species and some structures and sequences based on human immunoglobulin molecules. Humanized antibodies retain binding activity to the antigen while having reduced immunogenicity as compared to non-humanized antibodies.

The nanobody or the fusion protein mentioned herein is typically isolatable or recombinant. "Isolatable" means being capable of being identified and isolated and/or collected from a cell or cell culture where a polypeptide or protein is expressed. Typically, an isolated polypeptide (e.g., an isolated antibody or an isolated fusion protein) can be prepared through at least one purification step. "Isolated antibody" refers to an antibody that is substantially free of other antibodies or antigen-binding fragments with different antigen specificities (for example, an isolated antibody that specifically binds to PD-L1 is substantially free of antibodies that specifically bind to antigens other than PD-L1). "Recombinant" means that the antibody can be produced in an exogenous host cell using genetic recombination technology.

Herein, "comprise" or "include" means including the stated elements or steps but not excluding other elements or steps. Certainly, "comprise" or "include" also encompasses instances consisting of the stated elements or steps, unless otherwise stated. For example, when referring to comprising a particular sequence, it is also intended to encompass an antibody that consists of that particular sequence.

The "affinity" or "binding affinity" mentioned herein is to be understood in the general sense of the art and is used to reflect the strength and/or stability between an antigen and a binding site on an antibody or antigen-binding fragment.

"Specifically binding" or "specifically binding to" a particular antigen or epitope, or being "specific for" a particular antigen or epitope means being distinguished from non-specific interactions; such specific binding can be measured through some commonly used methods in the art. The ability of an antibody or antigen to bind can be measured through the enzyme-linked immunosorbent assay (ELISA) or other techniques familiar to those skilled in the art. For example, antigen-carrying cells can be detected by flow cytometry, and the competitive binding between the test antibody and the labeled antibody can be detected by measuring the positive rate of cells. Since the spatial structures of the antigens on the cell surface are more similar to the forms existing *in vivo*, the real situation can be better reflected through that method. According to a specific embodiment of the present invention, the nanobody provided has an EC50 value of ≤100 nM, ≤100 nM, ≤50 nM, ≤20 nM, ≤10 nM, ≤5 nM, ≤1 nM, ≤0.5 nM, ≤0.1 nM, ≤0.09 nM, ≤0.08 nM, ≤0.07 nM, ≤0.06 nM, ≤0.05 nM, ≤0.04 nM, ≤0.03 nM, ≤0.02 nM, ≤0.01 nM, or ≤0.001 nM. In addition, the binding activity of an antibody to an antigen can also be measured through surface plasmon resonance (SPR) technology or bio-layer interferometry (BLI) technology.

The names of amino acids used herein are indicated by one-letter or three-letter codes as is standard in the art. The "homology" of sequences mentioned herein refers to the degree to which proteins or polypeptides or nucleic acid sequences are identical when compared. The determination of homology can be accomplished in a variety of ways known in the art. For example, homology can be obtained by using publicly available software such as BLAST, ALIGN, BLAST-2, etc. The "conservative amino acid substitution" of a sequence mentioned herein refers to the replacement of an amino acid residue with a different amino acid residue that has a side chain with similar physiochemical properties. For example, conservative amino acid substitutions may be made between amino acid residues that have hydrophobic side chains (e.g., Met, Ala, Val, Leu, and Ile), between residues that have neutral hydrophilic side chains (e.g., Cys, Ser, Thr, Asn, and Gln), between residues that have acidic side chains (e.g., Asp and Glu), between amino acids that have basic side chains (e.g., His, Lys, and Arg), or between residues that have aromatic side chains (e.g., Trp, Tyr, and Phe). As is known in the art, conservative amino acid substitutions typically do not cause significant changes in the conformational structure of a protein; thus, the biological activity of the protein can be preserved.

The CDR sequences of the antibodies shown herein are obtained by analysis using existing databases, e.g., the IMGT database (Ehrenmann F., Kaas Q and Lefranc M.-P. Nucleic Acids Res., 38:D301-D307(2010); Ehrenmann, F., Lefranc, M.-P. Cold Spring Harbor Protoc., 6:737-749(2011)). Certainly, these sequences can also be obtained through Kabat (see, for example, U.S.Dept. of Health and Human Servies, "Sequences of Proteins of Immunological Interest" (1983)), Chothia (see, for example, J. Mol. Biol. 196:901-917(1987)), etc. It will be appreciated by those skilled in the art that differences in CDR sequences resulting from differences in database analysis methods are all within the protection scope of the present invention.

### Anti-PD-L1 Nanobody

The present invention provides an anti-PD-L1 nanobody, comprising a CDR1, a CDR2, and a CDR3, wherein (a) the CDR1 comprises a sequence selected from the group consisting of: SEQ ID NOs: 1, 5, and 9, or sequences that have one or two amino acid substitutions, deletions, or additions compared to the sequences of SEQ ID NOs: 1, 5, and 9; (b) the CDR2 comprises a sequence selected from the group consisting of: SEQ ID NOs: 2, 6, and 10, or sequences that have one or two amino acid substitutions, deletions, or additions compared to SEQ ID NOs: 2, 6, and 10; (c) the CDR3 comprises a sequence selected from the group consisting of SEQ ID NOs: 3, 7, 11, and 14, or sequences that have one, two, three, or four amino acid substitutions, deletions, or additions compared to the sequences of SEQ ID NOs: 3, 7, 11, and 14. For example, the amino acid deletions mentioned may be the deletion of two alanines (abbreviated as AA) or one alanine and one threonine (abbreviated as AT) from the N-terminus of the listed CDR3 sequence. For example, the amino acid additions mentioned may be the sequential connection of a methionine (abbreviated as M) and a glycine (abbreviated as G), or a methionine (M) and an arginine (abbreviated as R), etc., to the C-terminus of the listed CDR1 sequence.

The present invention provides an anti-PD-L1 nanobody, which is selected from: (1) an antibody that comprises a CDR1, which is the amino acid sequence set forth in SEQ ID NO: 1; a CDR2, which is the amino acid sequence set forth in SEQ ID NO: 2; and a CDR3, which is the amino acid sequence of SEQ ID NO: 3; or (2) an antibody that comprises a CDR1, which is the amino acid sequence set forth in SEQ ID NO: 5; a CDR2, which is the amino acid sequence set forth in SEQ ID NO: 6; and a CDR3, which is the amino acid sequence set forth in SEQ ID NO: 7; or (3) an antibody that comprises a CDR1, which is the amino acid sequence set forth in SEQ ID NO: 9; a CDR2, which is the amino acid sequence set forth in SEQ ID NO: 10; and a CDR3, which is the amino acid sequence set forth in SEQ ID NO: 11; or (4) an antibody that comprises a CDR1, which is the amino acid sequence set forth in SEQ ID NO: 1; a CDR2, which is the amino acid sequence set forth in SEQ ID NO: 2; and a CDR3, which is the amino acid sequence set forth in SEQ ID NO: 14. In some embodiments, the nanobody provided has a sequence that has one or two amino acid substitutions, deletions, or additions compared to the sequence set forth in SEQ ID NO: 1; has a sequence that has one or two amino acid substitutions, deletions, or additions compared to the sequence set forth in SEQ ID NO: 2; and has a sequence that has one, two, three, or four amino acid substitutions, deletions, or additions compared to the sequence set forth in SEQ ID NO: 3. In some embodiments, the nanobody provided has a sequence that has one or two amino acid substitutions, deletions, or additions compared to the sequence set forth in SEQ ID NO: 5; has a sequence that has one or two amino acid substitutions, deletions, or additions compared to the sequence set forth in SEQ ID NO: 6; and has a sequence that has one, two, three, or four amino acid substitutions, deletions, or additions compared to the sequence set forth in SEQ ID NO: 7. In some embodiments, the nanobody provided has a sequence that has one or two amino acid substitutions, deletions, or additions compared to the sequence set forth in SEQ ID NO: 9; has a sequence that has one or two amino acid substitutions, deletions, or additions compared to the sequence set forth in SEQ ID NO: 10; and has a sequence that has one, two, three, or four amino acid substitutions, deletions, or additions compared to the sequence set forth in SEQ ID NO: 11. In some embodiments, the nanobody provided has a sequence that has one or two amino acid substitutions, deletions, or additions compared to the sequence set forth in SEQ ID NO: 1; has a sequence that has one or two amino acid substitutions, deletions, or additions compared to the sequence set forth in SEQ ID NO: 2; and has a sequence that has one, two, three, or four amino acid substitutions, deletions, or additions compared to the sequence set forth in SEQ ID NO: 14.

The amino acid sequences of the CDRs mentioned are shown in Table 1 below.

**Table 1. The CDR sequences of different antibodies**

| Sequence No. | Amino acid sequence |
|---|---|
| SEQ ID NO:1 | GRSFSSSG |
| SEQ ID NO:2 | INSSGGDT |
| SEQ ID NOG | AAKEGGGPS SIPAIYDY |
| SEQ ID NO:5 | GFTFSSYA |
| SEQ ID NO:6 | INTGSEIV |
| SEQ ID NO:7 | ATGLVSAEHDGI |
| SEQ ID NO:9 | GRDFLTYG |
| SEQ ID NO: 10 | INWSGSMT |
| SEQ ID NO: 11 | AARRGAVTYASSNEYEH |
| SEQ ID NO: 14 | AAKEGGGPS STPAIFDY |

The present invention provides an anti-PD-L1 nanobody, comprising a variable region domain consisting of framework regions (FRs) and complementarity-determining regions (CDRs). The nanobody provided comprises framework regions (FRs) in addition to the CDR sequences mentioned. The framework regions (FRs) include an FR1, an FR2, an FR3, and an FR4. The framework regions (FRs) and CDR sequences are alternated to form the nanobody. In at least some embodiments, the FR1 is selected from the amino acid sequence set forth in SEQ ID NO: 16, or SEQ ID NO: 20, or SEQ ID NO: 23, or SEQ ID NO: 26. In some embodiments, the FR1 is selected from a sequence that has at least one, two, three, four, or five conservative amino acid substitutions compared to SEQ ID NO: 16, 20, 23, or 26. In at least some embodiments, the FR2 is selected from the amino acid sequence set forth in SEQ ID NO: 17, or SEQ ID NO: 21, or SEQ ID NO: 24. In some embodiments, the FR2 is selected from a sequence that has at least one, two, three, or four conservative amino acid substitutions compared to SEQ ID NO: 17, 21, or 24. In at least some embodiments, the FR3 is selected from the amino acid sequence set forth in SEQ ID NO: 18, or SEQ ID NO: 22, or SEQ ID NO: 25, or SEQ ID NO: 27. In some embodiments, the FR3 is selected from a sequence that has at least one, two, three, four, five, or six conservative amino acid substitutions compared to SEQ ID NO: 18, 22, 25, or 27. In at least some embodiments, the FR4 is selected from the amino acid sequence set forth in SEQ ID NO: 19, or a sequence that has one, two, or three conservative amino acid substitutions compared to the sequence set forth in SEQ ID NO: 19. The conservative amino acid substitutions mentioned for the FRs are preferably those that result in the formation of human framework regions.

The amino acid sequences of the framework regions are shown in Table 2 below.

**Table 2. Framework region sequences**

| Sequence No. | Amino acid sequence |
|---|---|
| SEQ ID NO:16 | QVQLUESGGGLUQAGGSLRLSCAAS |
| SEQ ID NO:17 | MGWFRQAPGKDREFVAT |
| SEQ ID NO:18 | YYRDSVEGRFTTSRDNAKNTMSLQMNDLKPEDTAVYYC |
| SEQ ID NO:19 | WGQGTQVTVSS |
| SEQ ID NO:20 | QVQLVESGGGLVQPGGSLSLSCVAS |
| SEQ ID NO:21 | MRWVRQAPGKEPEWVAG |
| SEQ ID NO:22 | VYANSVRGRFAISRDNARDTLFLQMNSLKPEDTAVYYC |
| SEQ ID NO:23 | EVQLVESGGGSVQTGGSLRLSCVAS |
| SEQ ID NO:24 | MGWFRQAPGKEREFVAS |
| SEQ ID NO:25 | YYADSVKGRFTISRDNAKNTVYLQMNSLKPGDTAVYIC |
| SEQ ID NO:26 | EVQLVESGGGLVQAGDSLRLSCTAS |
| SEQ ID NO:27 | YYADSVEGRFTTSRDNAKNTMSLQMNDLKPEDTAVYYC |

In at least some embodiments, the nanobody provided comprises framework regions (FRs) in addition to the CDRs. The framework regions (FRs) are selected from: (1) an FR1, which is the amino acid sequence set forth in SEQ ID NO: 16; an FR2, which is the amino acid sequence set forth in SEQ ID NO: 17; an FR3, which is the amino acid sequence set forth in SEQ ID NO: 18; and an FR4, which is the amino acid sequence set forth in SEQ ID NO: 19; or (2) an FR1, which is the amino acid sequence set forth in SEQ ID NO: 20; an FR2, which is the amino acid sequence set forth in SEQ ID NO: 21; an FR3, which is the amino acid sequence set forth in SEQ ID NO: 22; and an FR4, which is the amino acid sequence set forth in SEQ ID NO: 19; or (3) an FR1, which is the amino acid sequence set forth in SEQ ID NO: 23; an FR2, which is the amino acid sequence set forth in SEQ ID NO: 24; an FR3, which is the amino acid sequence set forth in SEQ ID NO: 25; and an FR4, which is the amino acid sequence set forth in SEQ ID NO: 19; or (4) an FR1, which is the amino acid sequence set forth in SEQ ID NO: 26; an FR2, which is the amino acid sequence set forth in SEQ ID NO: 24; an FR3, which is the amino acid sequence set forth in SEQ ID NO: 25; and an FR4, which is the amino acid sequence set forth in SEQ ID NO: 19; or (5) an FR1, which is the amino acid sequence set forth in SEQ ID NO: 16; an FR2, which is the amino acid sequence set forth in SEQ ID NO: 17; an FR3, which is the amino acid sequence set forth in SEQ ID NO: 27; and an FR4, which is the amino acid sequence set forth in SEQ ID NO: 19.

In at least some embodiments, the present invention provides an anti-PD-L1 nanobody, comprising the amino acid sequence set forth in SEQ ID NO: 4. In at least some embodiments, provided is an anti-PD-L1 nanobody, comprising the amino acid sequence set forth in SEQ ID NO: 8. In at least some embodiments, provided is an anti-PD-L1 nanobody, comprising the amino acid sequence set forth in SEQ ID NO: 12. In at least some embodiments, provided is an anti-PD-L1 nanobody, comprising the amino acid sequence set forth in SEQ ID NO: 13. In at least some embodiments, provided is an anti-PD-L1 nanobody, comprising the amino acid sequence set forth in SEQ ID NO: 15.

The amino acid sequences of the anti-PD-L1 nanobody provided are shown in Table 3 below.

**Table 3. Amino acid sequences**

| No. | Amino acid sequence |
|---|---|
| SEQ ID NO:4 | QVQLVESGGGLVQAGGSLRLSCAASGRSFSSSGMGWFRQAPG KDREFVATINSSGGDTYYRDSVEGRFTTSRDNAKNTMSLQMND LKPEDTAVYYCAAKEGGGPSSIPAIYDYWGQGTQVTVSS |
| SEQ ID NO:8 | QVQLVESGGGLVQPGGSLSLSCVASGFTFSSYAMRWVRQAPGK EPEWVAGINTGSEIVVYANSVRGRFAISRDNARDTLFLQMNSLK PEDTAVYYCATGLVSAEHDGIWGQGTQVTVSS |
| SEQ ID NO:12 | EVQLVESGGGSVQTGGSLRLSCVASGRDFLTYGMGWFRQAPG KEREFVASINWSGSMTYYADSVKGRFTISRDNAKNTVYLQMNS LKPGDTAVYICAARRGAVTYASSNEYEHWGQGTQVTVSS |
| SEQ ID NO:13 | EVQLVESGGGLVQAGDSLRLSCTASGRDFLTYGMGWFRQAPG KEREFVAS1NWSGSMTYYADSVKGRFTISRDNAKNTVYLQMN SLKPGDTAVYICAARRGAVTYASSNEYEHWGQGTQVTVSS |
| SEQ ID NO:15 | QVQLVESGGGLVQAGGSLRLSCAASGRSFSSSGMGWFRQAPG KDREFVATINSSGGDTYYADSVEGRFTTSRDNAKNTMSLQMND LKPEDTAVYYCAAKEGGGPSSIPAIFDYWGQGTQVTVSS |

In at least some embodiments, the anti-PD-L1 nanobody has an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology compared to the amino acid sequence set forth in SEQ ID NO: 4, SEQ ID NO: 8, SEQ ID NO: 12, SEQ ID NO: 13, or SEQ ID NO: 15. The anti-PD-L1 nanobody provided has at least one conservative amino acid substitution, e.g., 1 conservative amino acid substitution, 2 conservative amino acid substitutions, 3 conservative amino acid substitutions, 4 conservative amino acid substitutions, 5 conservative amino acid substitutions, 6 conservative amino acid substitutions, 7 conservative amino acid substitutions, 8 conservative amino acid substitutions, 9 conservative amino acid substitutions, or 10 conservative amino acid substitutions, compared to the amino acid sequence set forth in SEQ ID NO: 4, SEQ ID NO: 8, SEQ ID NO: 12, SEQ ID NO: 13, or SEQ ID NO: 15. The conservative amino acid substitutions mentioned preferably occur in the framework regions.

The anti-PD-L1 nanobody provided may also be a humanized nanobody. The humanized nanobody is less immunogenic while retaining the binding activity of the nanobody to PD-L1. In at least some embodiments, the humanized nanobody provided retains the CDR amino acid sequences and has humanized framework region sequences in place of the framework regions (FRs). In at least some embodiments, the framework region (FR) sequences of the humanized nanobody provided comprise partially humanized framework region sequences. According to a specific embodiment, the partially humanized framework region sequences mentioned may be obtained by replacing the framework region sequences with humanized framework region sequences and then back-mutating key amino acids that affect the structure and function of the antibody. The humanized nanobody may be obtained through methods commonly used in the art (e.g., complementarity-determining region grafting). Human framework sequences may be obtained from published human sequences. For example, human FR templates with the greatest homology are found through database alignment and computer homology modeling; key residues of the FRs that need to be back-mutated are determined by comprehensive consideration; and a humanized antibody with high affinity is obtained. These sequences are usually set forth in commonly used databases, such as the PDB protein structure database, IMGT, Genebank, etc. The humanized nanobody may be further back-mutated to retain some of the properties of the original sequence, such as affinity and physiological activity. These back mutations occur mostly in the framework regions FR2 and FR3. For example, a back mutation occurs at position 2, 10, 11, 12, or the like of the region FR2. For example, a back mutation occurs at position 10, 12, 13, 21, 29, 32, or the like of the region FR3.

In at least some embodiments, the humanized nanobody is set forth in SEQ ID NO: 28, SEQ ID NO: 29, SEQ ID NO: 30, SEQ ID NO: 31, or SEQ ID NO: 32. The humanized nanobody provided has low immunogenicity and a high affinity for PD-L1.

**Table 4. Humanized amino acid sequences.**

| Sequence No. | Amino acid sequence |
|---|---|
| SEQ ID NO:28 | QVQLVESGGGVVQPGGSLRLSCAASGRSFSSSGMGWFRQAPG KGREFVATINSSGGDTYYRDSVEGRFTTSRDNSKNTMYLQMN SLRAEDTAVYYCAAKEGGGPSSIPAIYDYWGQGTLVTVSS |
| SEQ ID NO:29 | QVQLVESGGGVVQPGGSLRLSCAASGFTFSSYAMRWVRQAPG KGLEWVAG1NTGSE1VVYANSVRGRFTISRDNSRDTLYLQMNS LRAEDTAVYYCATGLVSAEHDGIWGQGTQVTVSS |
| SEQ ID NO:30 | EVQLVESGGGLVQPGGSLRLSCAASGRDFLTYGMGWFRQAPG KGREFVASINWSGSMTYYADSVKGRFTISRDNAKNTVYLQMN SLKAEDTAVYICAARRGAVTYASSNEYEHWGQGTLVTVSS |
| SEQ ID NO:31 | EVQLVESGGGLVQPGGSLRLSCTASGRDFLTYGMGWFRQAPG KELEFVASINWSGSMTYYADSVKGRFTISRDNAKNTVYLQMN SLRAEDTAVYICAARRGAVTYASSNEYEHWGQGTQVTVSS |
| SEQ ID NO:32 | EVQLVESGGGLVQPGGSLRLSCAASGRDFLTYGMGWFRQAPG KGLEFVASTNWSGSMTYYADSVKGRFTTSRDNAKNSVYLQM NSLRAEDTAVYICAARRGAVTYASSNEYEHWGQGTQVTVSS |

The present invention further provides an anti-PD-L1 nanobody, selected from: i) an antibody that has the amino acid sequence set forth in SEQ ID NO: 4, or SEQ ID NO: 8, or SEQ ID NO: 12, or SEQ ID NO: 13, or SEQ ID NO: 15, or SEQ ID NO: 28, or SEQ ID NO: 29, or SEQ ID NO: 30, or SEQ ID NO: 31, or SEQ ID NO: 32; or ii) an antibody that has an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology compared to i).

### Fusion Protein

The present invention further provides a fusion protein, wherein the fusion protein comprises: a first domain, which is the anti-PD-L1 nanobody described above; and a second domain, which is a functional protein different from the anti-PD-L1 nanobody. The first domain and the second domain may be directly linked or may be linked by a linker. The fusion protein has the characteristics of an anti-PD-L1 antibody and the activity of a functional protein. It can be not only used for preparing immune targeting drugs but also widely used for immune-diagnosis, antibody purification, quantitative analysis of antibodies and antigens, etc.

Herein, useable linkers are those that are commonly used in the art and may be some oligopeptides or polypeptides. These oligopeptides or polypeptides may be any amino acid sequences that are capable of providing flexibility. Linkers include, but are not limited to, the group consisting of GS, SG, GGS, GSG, SGG, GGG, GGGS, SGGG, GGGGS, GGGGGSGS, GGGGSGS, GGGGSGGS, GGGGSGGGGSGGGGS, GGGGSGGGGS, GGGGSGGGGSGGGGSGGGGS, GGGGSGGGGSGGGGSGGGGSGGGGS, GGGGSGGGGSGGGGSGGGGSGGGSGGGS, etc.

In at least some embodiments, the second domain may be linked to the C-terminal (carboxy-terminal) amino acid of the first domain. The second domain may be one or more functional proteins; after the C-terminus of the first domain is linked to one functional protein molecule, it may also be linked to one or more functional protein molecules if desired and can bind to one of the functional protein molecules through the C-terminal amino acid or the N-terminal amino acid (the amino-terminus).

The second domain may be an immunoglobulin Fc region, for example, a human immunoglobulin Fc region. The nanobody lacks an Fc domain and thus has no ADCC or CDC activity. The protein fusion of the nanobody and the immunoglobulin Fc region can enhance the ADCC or CDC function of the nanobody and extend the half-life of the drug in the body. The second domain may also be a cytokine. The cytokine mentioned includes, but is not limited to, IL-2, IL-3, IL-4, IL-5, IL-6, IL-7, IL-8, IL-9, IL-10, IL-12, IL-13, IL-15, IL-14, IL-18, IL-21, IFN-γ, TNF, TGF-β, GM-CSF, etc. One or more of these cytokines may be linked to the first domain to form a fusion protein. In at least some embodiments, the cytokine is linked to the C-terminal amino acid of the first domain. The second domain may also be an antigen-targeting protein different from PD-L1. The nanobody is small in size and high in stability, and can be conveniently combined with other antigen-targeting proteins to form bispecific antibodies or multi-specific antibodies; for example, it can be combined with antigen-targeting proteins such as anti-PD-1, anti-PD-L1, anti-EGFR, anti-c-Met, anti-TIM-3, anti-LAG-3, anti-CD-47, anti-TIGIT, anti-CD-39, anti-CD-73, anti-41BB, anti-OX40, anti-CD-3, anti-CD-40, anti-GARP-TGF-β Complex, anti-TGF-β, anti-GARP, anti-Her2, anti-Her3, anti-CTLA-4, anti-GARP-TGF-β complex, etc. to prepare bi- or multi-specific antibodies and to prepare antibodies with more antigen-targeting functions. The nanobody may be conjugated to a diagnostic agent (e.g., a radionuclide, an optical tracer, or the like) to enable specific tumor imaging.

The fusion protein may be prepared through methods commonly used in the art. For example, the fusion proteins may be prepared using genetic engineering technology. Genes encoding the first domain and the second domain are ligated by an adapter sequence, and the resulting ligation product is cloned into a vector and expressed through a prokaryotic or eukaryotic expression system.

The anti-PD-L1 nanobody or the fusion protein mentioned herein is substantially pure. The "substantially pure" means that the purity of the anti-PD-L1 nanobody produced can reach 90% or more, 91% or more, 92% or more, 93% or more, 94% or more, 95% or more, 96% or more, 97% or more, 98% or more, 99% or more, 99.5% or more, etc.

### Antibody Conjugate

The present invention further provides an antibody conjugate, wherein the antibody conjugate comprises the anti-PD-L1 nanobody or the fusion protein; and a functional small molecule linked to the anti-PD-L1 nanobody or the fusion protein. The functional small molecule mentioned may be a developed or undeveloped small-molecule drug. The nanobody has good solubility and high stability and is suitable for chemical conjugation with a small-molecule drug. The nanobody and the small-molecule drug are conjugated to form a nanobody-drug conjugate, which can enhance the therapeutic effect of the anti-tumor drug and reduce adverse effects. For example, a small molecule such as a toxin, a chemotherapeutic drug, a photosensitizer, or the like may be conjugated to the nanobody by a linker. Certainly, the antibody may also be conjugated with a Protac molecule or the like to obtain an antibody-Protac conjugate; for example, the antibody is linked to a protease-targeting ligand (such as an E3 ligase ligand) by a linker, so that the target protein is drawn closer to E3 ubiquitin ligase in cells and specifically degraded through the ubiquitin-proteasome pathway.

### Isolated Polynucleotide

The present invention further provides an isolated polynucleotide, wherein the isolated polynucleotide encodes the anti-PD-L1 nanobody described above or the fusion protein. The isolated polynucleotide may be DNA, RNA, cDNA, or the like. The polynucleotide sequence that encodes the anti-PD-L1 nanobody or the fusion protein may be obtained according to a method conventionally used by those skilled in the art.

### Construct

The present invention further provides a construct, comprising the isolated polynucleotide mentioned herein. The construct can be obtained by a variety of methods commonly used in the art, such as *in vitro* recombinant DNA technology, DNA synthesis technology, *in vivo* recombinant technology, etc.; for example, the construct can be formed by inserting the polynucleotide into the multiple cloning site of an expression vector. The construct may comprise, if desired, various operators such as a promoter, a terminator, a marker gene, etc., and these operators are operably ligated to the polynucleotide. Promoters are generally used to provide a signal for starting transcription. The lactose promoter (Lac), the Trp promoter, the Tac promoter, or the PL and PR promoters of phages may be selected, if desired. Terminators provide a signal for terminating transcription during transcription. Marker genes on constructs are often used for screening. Certainly, an enhancer may be included, if desired, to enhance protein expression. The expression vector is not particularly limited and may be some of the commercially available expression vectors or may be an expression vector that is artificially modified if desired, e.g., a plasmid, a phage, a virus, or the like. The virus may be a plant cell virus, a mammalian cell virus, or the like. The construct can express the antibody or protein *in vitro* and can also be transferred into a cell to express the antibody or protein.

### Host Cell

The present invention further provides a host cell, containing the isolated polynucleotide described above or the construct described above. Any cell suitable for antibody or protein expression of the polynucleotide or the construct may be used as a host cell. The host cell may be a prokaryotic cell, such as a bacterial cell; or a eukaryotic cell, such as a yeast cell, a mammalian cell, or the like. Host cells commonly used may be yeast cells, CHO, HEK-293, COS cells, Drosophila S2, or Sf9 insect cells. The host cell comprising the polynucleotide, or the construct may be obtained by methods commonly used in the art, such as microinjection, electroporation, chemical transfection, virus-mediated transformation, etc.

### Method for Producing Nanobody or Fusion Protein

The present invention further provides a method for producing a nanobody or a fusion protein, comprising culturing the host cell mentioned in the present invention; and collecting the nanobody or the fusion protein. After the collected nanobody or fusion protein is purified, a substantially pure nanobody or fusion protein product can be obtained. The "substantially pure" means that the purity of the anti-PD-L1 nanobody produced can reach 90% or more, 91% or more, 92% or more, 93% or more, 94% or more, 95% or more, 96% or more, 97% or more, 98% or more, 99% or more, 99.5% or more, etc.

### Pharmaceutical Composition

The present invention further provides a pharmaceutical composition, comprising the anti-PD-L1 nanobody, or the fusion protein, or the antibody conjugate; and a pharmaceutically acceptable carrier.

The pharmaceutically acceptable carrier mentioned is acceptable to the subject at the dosage or concentration employed. The pharmaceutically acceptable carrier includes, but is not limited to buffers or salts, such as Tris, phosphate, citrate, sodium acetate, citric acid, sodium citrate, and other organic acids; antioxidants, such as ascorbic acid; preservatives, such as benzalkonium chloride; amino acids, such as histidine, histidine hydrochloride, glycine, glutamine, asparagine, arginine, or lysine. Pharmaceutical formulations for use in the body of a subject are generally sterile. Sterile pharmaceutical formulations may be obtained by methods commonly used in the art, for example, by filtration through sterile filter membranes. One skilled in the art may select suitable pharmaceutically acceptable carriers based on the dosage forms in which the pharmaceutical composition is to be prepared, to prepare different dosage forms. For example, the pharmaceutical composition may be prepared in various dosage forms such as an injection, a lyophilizate, a tablet, etc.

### Kit

The present invention further provides a kit, wherein the kit comprises the nanobody described above, or the fusion protein described above, or the antibody conjugate. The kit may further comprise, if desired, a container, a buffer, and controls such as a positive control and a negative control. One skilled in the art may make a corresponding selection if desired. Accordingly, the kit may further comprise instructions for use to facilitate the operation and use of the kit by those skilled in the art.

### Method for Preventing and/or Treating Disease

The present invention further provides a method for preventing and/or treating disease, comprising administering to a subject a prophylactically and/or therapeutically effective amount of the anti-PD-L1 nanobody, or the fusion protein, or the antibody conjugate described above.

The "therapeutically effective amount" mentioned can result in a reduction in the severity of the symptoms of the disease, an increase in the frequency and duration of the asymptomatic phase of the disease or prevent reductions in the suffering caused by the disease. The "prophylactically effective amount" is usually lower than the therapeutically effective amount. Compared with a subject who has not been treated with the anti-PD-L1 nanobody provided, a subject who has been treated with the anti-PD-L1 nanobody provided shows an inhibition rate against *in vivo* cells of 10% or more, 15% or more, 20% or more, 25% or more, 30% or more, 40% or more, 45% or more, 50% or more, 55% or more, 60% or more, 65% or more, 70% or more, 75% or more, 80% or more, 85% or more, or even 90% or more, or 95% or more. The subject mentioned may be an animal or a human. For example, the subject mentioned may be a mammal, including cows, sheep, mice, horses, etc.

The anti-PD-L1 nanobody or the fusion protein or the antibody conjugate can be used for treating various diseases, especially cancers, including but not limited to non-small cell lung cancer (NSCLC), melanoma, urothelial cancer, hepatocellular carcinoma, renal cell carcinoma, classical Hodgkin lymphoma, cervical cancer, head and neck squamous cell carcinoma, bladder cancer, lung cancer, nasopharyngeal carcinoma, gastric cancer, esophageal cancer, colorectal cancer, breast cancer, ovarian cancer, etc. Certainly, the anti-PD-L1 nanobody or the fusion protein provided can also be used in combination with many other antibodies to prevent or treat disease. For example, the anti-PD-L1 nanobody or the fusion protein provided can be used in combination with a PD-1 antibody, a CTLA-4 antibody, an EGFR antibody, a c-Met antibody, a CD-40 antibody, a CD-3 antibody, a CD70 antibody, a TIGIT antibody, a CD-39 antibody, an OX40 antibody, a TGFβ antibody, a GARP antibody, a Her2 antibody, a c-Met antibody, a LAG-3 antibody, a Tim-3 antibody, a 4-1BB antibody, or the like to enhance the therapeutic effect on cancer. The antibody provided can be administered at a therapeutically effective dose of about 0.01 mg/kg to about 100 mg/kg, e.g., 0.1 mg/kg to 100 mg/kg, 1 mg/kg to 100 mg/kg, 5 mg/kg to 90 mg/kg, 10 mg/kg to 80 mg/kg, 10 mg/kg to 70 mg/kg, 10 mg/kg to 60 mg/kg, 10 mg/kg to 50 mg/kg, 10 mg/kg to 40 mg/kg, 10 mg/kg to 35 mg/kg, 15 mg/kg to 30 mg/kg, 20 mg/kg to 30 mg/kg, or 15 mg/kg to 25 mg/kg. In certain embodiments, the dose administered may vary depending on the course of treatment. In some embodiments, the dose administered may vary depending on the subject's response during treatment.

The anti-PD-L1 nanobody or the fusion protein provided can also be combined with various clinical treatment regimens, such as chemotherapy, radiotherapy, anti-angiogenesis therapy, surgery, etc., to improve the curative effect on tumors. For example, larger tumors can be fragmented by radiotherapy, and then tiny tumors are encircled and intercepted by immunotherapy, so that killing of tumor cells can be maximized.

The present invention provides use of the anti-PD-L1 nanobody or the fusion protein or the antibody conjugate in the preparation of a medicament or a kit. The anti-PD-L1 nanobody or the fusion protein or the antibody conjugate can be used for preparing a medicament for preventing or treating various diseases. The anti-PD-L1 nanobody or the fusion protein or the antibody conjugate can also be used for preparing a kit and be used as a reagent component in the kit for diagnosis of the specific antigen.

The examples of the present invention are described in detail with reference to the accompanying drawings. It should be noted that these examples are intended to explain the present invention and facilitate the understanding of the present invention by those skilled in the art and should not be construed as limiting the protection scope of the present invention. Unless otherwise indicated, the experimental methods, assay methods, preparation methods, etc. disclosed in the present invention were all performed using molecular biology, biochemistry, analytical chemistry, cell culture, and recombinant DNA technologies conventional in the art, and conventional technologies in related arts. These technologies are well described and explained in the literature.

### Example 1

An anti-PD-L1 nanobody library was obtained by an alpaca immunization method, and meanwhile screening and identification were carried out to obtain candidate nanobodies.

A fusion protein composed of the human PD-L1 protein ectodomain sequence and the human immunoglobulin Fc region sequence was mixed with Freund's adjuvant, and the mixture was emulsified and used to immunize healthy alpacas, stimulating the expression of antigen-specific nanobodies by B cells. Then blood was collected from the alpacas, and lymphocytes were separated. Total RNA was extracted using the Trizol method and reverse-transcribed into cDNA. The resulting cDNA was subjected to PCR amplification, and VHH antibody gene fragments were obtained. Competent yeast cells were transformed with the VHH gene fragments together with a yeast display vector by electroporation. The fragments and the vector were ligated by homologous recombinases of yeast to form complete plasmids. A yeast transformant library with a high insertion rate and excellent diversity was constructed and stably passaged in a nutritionally deficient culture medium.

Antibody-expressing yeast cells and magnetic beads enriched with the target protein antigen were co-incubated. After several enrichment cultures, the products of magnetic bead enrichment were identified using a flow cytometer and sorted. Through several rounds of screening, 5 positive clones with high expression were obtained and designated antibody 1-antibody 5. The amino acid sequence of antibody 1 is set forth in SEQ ID NO: 4, as determined by sequencing, and analysis with the IMGT/DomainGapAlign tool showed that its CDRs are SEQ ID NOs: 1, 2, and 3, respectively. The amino acid sequence of antibody 2 is set forth in SEQ ID NO: 8, and its CDRs are SEQ ID NOs: 5, 6, and 7, respectively. The amino acid sequence of antibody 3 is set forth in SEQ ID NO: 12, and its CDRs are SEQ ID NOs: 9, 10, and 11, respectively. The amino acid sequence of antibody 4 is set forth in SEQ ID NO: 13, and its CDRs are SEQ ID NOs: 9, 10, and 11, respectively. The amino acid sequence of antibody 5 is set forth in SEQ ID NO: 15, and its CDRs are SEQ ID NOs: 1, 2, and 14, respectively.

### Example 2

The different antibodies obtained were expressed using a mammalian cell system and purified, and antibodies with at least 90% or higher purity were obtained. Then the binding activity of the different antibodies to different antigens was determined. Atezolizumab (Tecentriq, Roche) was used as a positive control. The positive controls referred to in the following examples were all atezolizumab.

### (1) FACS assay for binding activity of antibodies to hPD-L1

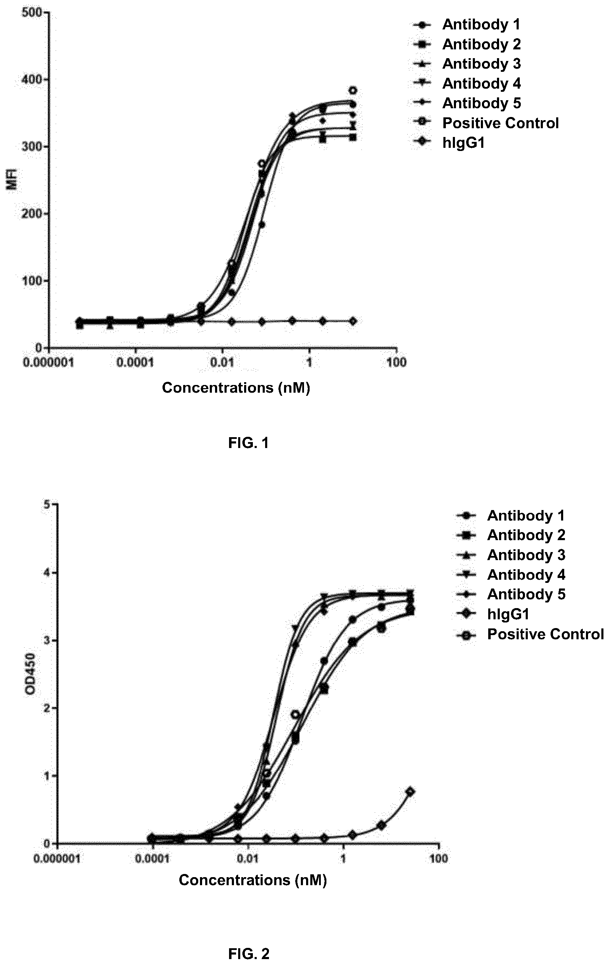

The affinities of the different antibodies for the antigen were measured using a flow cytometer. MDA-MB-231 cells (ATCC) that endogenously express the PD-L1 antigen were added to a 96-well plate at 1 × 10⁵ cells/well. Antibody samples with different concentrations (the initial concentration was 50 nM, and 5-fold gradient dilution was performed) were then added, and the plate was incubated at 4 °C for 30 minutes. A fluorescently labeled goat anti-human IgG secondary antibody (manufacturer: Abcam) was then added to detect antibodies bound to the cell surfaces. Antibody-antigen binding dose response curves were generated using geometric values, and four-parameter raw data were plotted using the Graphpad Prism V6.0 software. The EC50 values for the binding of the antibodies to the antigen were determined, and the results are shown in Table 5. The mean fluorescence intensity (MFI) values obtained as a function of the antibody concentration are shown in FIG. 1.

**Table 5**

| | EC50 (nM) |
|---|---|
| Antibody 1 | 0.089 |
| Antibody 2 | 0.031 |
| Antibody 3 | 0.042 |
| Antibody 4 | 0.039 |
| Antibody 5 | 0.053 |
| hIgG 1 | No binding |
| Positive control | 0.059 |

The experimental results show that: antibodies 1 to 5 showed high binding activity at the cellular level, similar to the positive control.

### (2) ELISA assay for binding activity of antibodies to cynomolgus monkey PD-L1 (cyno PD-L1)

A 96-well plate was coated with 1 µg/mL Cynomolgus/Rhesus macaque PD-L1-hFc protein (manufacturer: ACRO Biosystems) and incubated at 4 °C overnight. The plate was washed with wash buffer and then blocked with a PBS solution containing 2% BSA for 60 minutes. After a wash with wash buffer, different concentrations of the antibodies to be tested (the initial concentration was 100 nM, and 4-fold gradient dilution was performed) were added, and the plate was incubated at 37 °C for 120 minutes. The plate was washed with wash buffer, and an HRP-labeled goat anti-human secondary antibody (diluted at 1:10,000, manufacturer: Jackson) was then added. The plate was incubated at 37 °C for 60 minutes. The plate was washed with wash buffer, and TMB substrate solution was added at 100 µL/well. After 10-15 minutes of reaction at room temperature, 100 µL of 2 M hydrochloric acid stop solution was added to stop the reaction. OD450 values were measured with a microplate reader, and EC50 values were calculated and are shown in Table 6. FIG. 2 shows the OD450 values measured at different antibody concentrations.

**Table 6**

| | EC50 (nM) |
|---|---|
| Antibody 1 | 0.133 |
| Antibody 2 | 0.136 |
| Antibody 3 | 0.040 |
| Antibody 4 | 0.034 |
| Antibody 5 | 0.034 |
| hIgG1 | No binding |
| Positive control | 0.094 |

It can be seen from the results that antibodies 1 to 5 can all bind to Cyno PD-L1 and exhibited high binding activity.

### (3) ELISA assay for binding activity of antibodies to mouse PD-L1

A 96-well plate was coated with 1 µg/mL mouse PD-L1-hFc protein (manufacturer: ACRO Biosystems) and incubated at 4 °C overnight. The plate was washed with wash buffer and blocked with a PBS solution containing 2% BSA for 60 minutes. After a wash with wash buffer, different concentrations of the antibodies to be tested (the initial concentration was 100 nM, and 4-fold gradient dilution was performed) were added, and the plate was incubated at 37 °C for 120 minutes. The plate was washed with wash buffer, and an HRP-labeled goat anti-human secondary antibody (diluted at 1:10,000) was then added. The plate was incubated at 37 °C for 60 minutes. The plate was washed with wash buffer, and TMB substrate solution was added at 100 µL/well. After 10-15 minutes of reaction at room temperature, 100 µL of 2 M hydrochloric acid stop solution was added to stop the reaction, and OD450 values were measured with a microplate reader.

The experimental results show that: none of the antibodies prepared exhibited binding activity to mouse PD-L1.

### Example 3

In example 3, the blocking activity of the antibodies against PD-1/PD-L1 or CD-80/PD-L1 was determined.

### (1) FACS assay for blocking activity of antibodies against PD-1/PD-L1

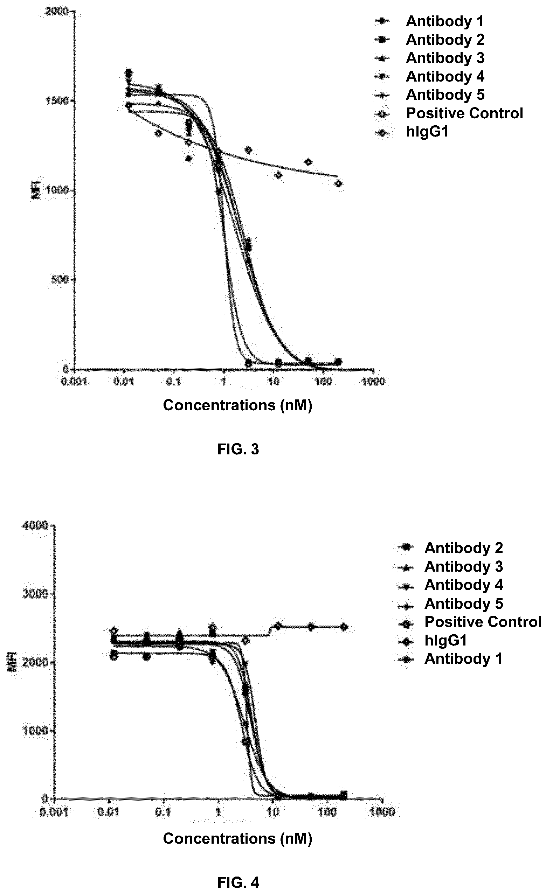

The blocking of PD-1 and its ligand PD-L1 by the antibodies was measured using a method based on competitive flow cytometry. A 293T-PD-1 cell line that overexpresses PD-1 (manufacturer: Kyinno Biotechnology (Beijing) Co., Ltd.) was thawed, and the cells were added to a 96-well plate at 1 × 10⁵ cells/well. Antibody dilutions with different concentrations (the initial working concentration was 200 nM, and 4-fold dilution was performed) were mixed with a PD-L1-Biotin dilution (2 µg/mL, 50 µL/well), and the mixtures were incubated at room temperature for 30 minutes. Then the mixtures were added to the cells at 100 µL/well and well mixed. The plate was incubated at 4 °C for 60 minutes, then washed with PBS containing 2% FBS, and centrifuged at 1200 rpm for 4 minutes, and the supernatants were discarded. PE-labeled streptavidin was added at 100 µL/well, and the plate was incubated in the dark for 30 minutes. After the incubation, the plate was washed with PBS containing 2% FBS. 120 µL of FACS buffer was added to resuspend the cells, and the plate was tested on the instrument. Live cells were gated based on FSC/SSC and their mean fluorescence values were measured. Antibody-antigen blocking reaction curves were generated using geometric values, and four-parameter plots were generated using the Graphpad Prism V6.0 software. The IC50 values were determined and are shown in Table 7 below. FIG. 3 shows the mean fluorescence intensity (MFI) results obtained as a function of the antibody concentration.

**Table 7**

| | IC50 (nM) |
|---|---|
| Antibody 1 | 1.024 |
| Antibody 2 | 2.077 |
| Antibody 3 | 1.678 |
| Antibody 4 | 2.103 |
| Antibody 5 | 2.536 |
| hIgG1 | No blocking |
| Positive control | 1.01 |

The experimental results show that the antibodies prepared can all block the binding of the antigen to its ligand.

### (2) FACS assay for blocking activity of antibodies against CD-80/PD-L1

The blocking of PD-L1 and CD-80 by the antibodies was measured using a method based on competitive flow cytometry. A 293T-PD-L1 cell line that overexpresses PD-L1 (manufacturer: Kyinno Biotechnology (Beijing) Co., Ltd.) was thawed, and the cells were added to a 96-well plate at 1 × 10⁵ cells/well. Antibody dilutions with different concentrations (the initial working concentration was 200 nM, and 4-fold dilution was performed) were added to the cells, and the plate was incubated at 4 °C for 60 minutes. After the incubation, the plate was washed with PBS containing 2% FBS and centrifuged at 1200 rpm for 4 minutes, and the supernatants were discarded. Then human-CD80-mFc (2 µg/mL) was added at 100 µL/well, and the plate was incubated in the dark at 4 °C for 30 minutes. After the incubation, the plate was washed twice with PBS containing 2% FBS. A fluorescently labeled goat anti-mouse secondary antibody (manufacturer: Abcam) diluted at 1:200 was added at 100 µL/well, and the plate was incubated in the dark at 4 °C for 30 minutes. After the incubation, the plate was washed twice with PBS containing 2% FBS and tested on a flow cytometer. The mean fluorescence values were measured. Antibody-antigen blocking reaction curves were generated using geometric values, and four-parameter plots were generated using the Graphpad Prism V6.0 software. The IC50 values were determined and are shown in Table 8 below. FIG. 4 shows the mean fluorescence intensity (MFI) results obtained as a function of the antibody concentration.

**Table 8**

| Sample No. | IC50 (nM) |
|---|---|
| Antibody 1 | 3.954 |
| Antibody 2 | 3.355 |
| Antibody 3 | 4.146 |
| Antibody 4 | 4.757 |
| Antibody 5 | 2.979 |
| Positive control | 2.676 |
| hIgG1 | NA |

The experimental results show that the antibodies provided can all well block the binding of antigen-human CD-80.

### Example 4

In Example 4, the blocking of the PD-1/PD-L1 Pathway by the antibodies was studied through a reporter gene experiment. The experiment consists of two genetically engineered cell lines: Jurkat-PDl-CD3zeta-NFAT-Luc2 effector cells (manufacturer: Kyinno Biotechnology (Beijing) Co., Ltd.), in which a fusion protein consisting of PD-1 ECD and CD-3zeta is stably expressed in Jurkat cells and a luciferase reporter gene driven by the factor of activated T-cells (NFAT) is inserted; and 293T-hPD-L1 cells (manufacturer: Kyinno Biotechnology (Beijing) Co., Ltd.), i.e., 293T cells that express human PD-L1. When the two types of cells are co-cultured, the interaction of PD-1/PD-L1 serves as a first signal, and the intracellular CD3zeta chain serves as a second signal; activation signals are transmitted inward, enabling the expression of the NFAT-driven luciferase reporter gene, which results in the emission of fluorescent light. When a PD-L1 antibody is added, the PD-1/PD-L1 binding is blocked, the transmission of activation signals is suppressed, and the luciferase reporter gene cannot be expressed.

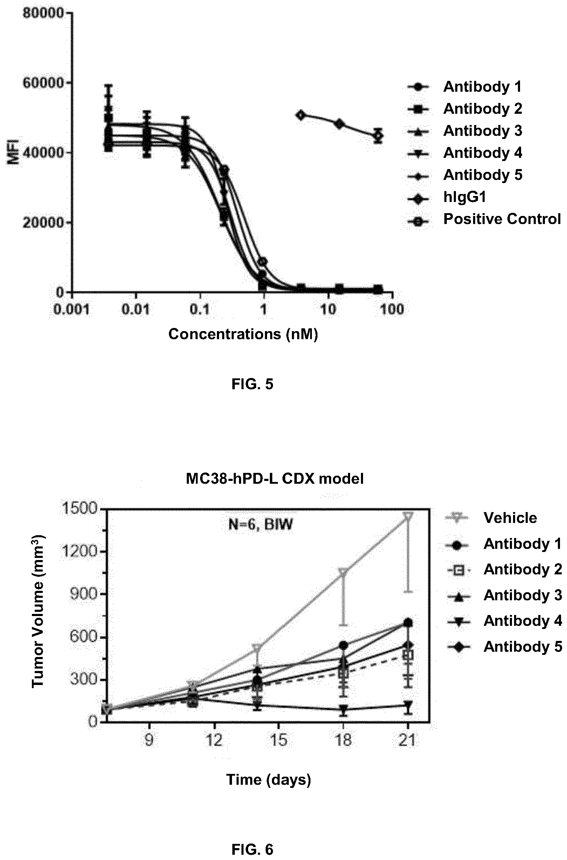

293T-hPD-L1 cells were added to a 96-well plate at 2 × 10⁴ cells/well, and Jurkat-PD1-CD3zeta-NFAT-Luc2 effector cells were then added to the 96-well plate at 2 × 10⁴ cells/well. Then different concentrations of the antibodies to be tested (the initial concentration was 60 µg/mL, and 4-fold gradient dilution was performed) were added, and the plate was incubated at 37 °C overnight. The luciferase substrate ONE-Glo^{™} Luciferase Assay system assay reagent (Promega) was added at 100 µL/well, and the plate was incubated in the dark for 5 minutes. The fluorescent signals in the 96-well plate were measured with a microplate reader. Four-parameter curves were generated with the y-axis representing the relative fluorescence value and the x-axis representing the antibody sample concentration. The curves were analyzed using the GraphPad Prism software, and the IC50 values for the antibody samples were obtained. FIG. 5 shows the mean fluorescence intensity results obtained as a function of the antibody concentration.

**Table 9**

| Antibody No. | IC50 (nM) |
|---|---|
| Antibody 1 | 0.378 |
| Antibody 2 | 0.200 |
| Antibody 3 | 0.211 |
| Antibody 4 | 0.260 |
| Antibody 5 | 0.305 |
| Positive control | 0.489 |
| hIgG1 | NA |

It can be seen from the results that antibodies 1 to 5 all exhibited blocking activity.

### Example 5

Humanized antibodies were obtained by the CDR-grafting method, which fulfills the purpose of humanization by dividing the amino acid sequences of an antibody and a template into FRs and CDRs in the same way and then grafting the CDRs onto the FRs of the template so that the CDRs that determine the specificity of the antibody are combined with the framework regions of a human antibody. Meanwhile, in order to avoid reductions in the affinity of the antibodies, key amino acid residue positions were obtained by calculations through computer simulation and other technologies, and a back mutation method was used to ensure the affinities of the humanized antibodies.

Finally, the sequence of humanized antibody 1 was numbered antibody 1hu and the amino acid sequence is set forth in SEQ ID NO: 28. The sequence of humanized antibody 2 was numbered antibody 2hu and the amino acid sequence is set forth in SEQ ID NO: 29. The sequence of humanized antibody 3 was numbered antibody 3hu and the amino acid sequence is set forth in SEQ ID NO: 30. One of the sequences of humanized antibody 4 was numbered antibody 4hu1 and the amino acid sequence is set forth in SEQ ID NO: 31; the other sequence was numbered antibody 4hu2 and the amino acid sequence is set forth in SEQ ID NO: 32.

### Example 6

In Example 6, the activity of the humanized antibodies prepared in Example 5 was measured.

### 1. FACS assay for binding activity of humanized antibodies to human PD-L1

The binding activity of the humanized antibodies to PD-L1 was measured by flow cytometry. An MC38-hPD-L1 cell line that overexpresses human PD-L1 (manufacturer: Kyinno Biotechnology (Beijing) Co., Ltd.) was thawed, and the cells were added to a 96-well plate at 1 × 10⁵ cells/well. Different concentrations of the antibodies to be tested (the initial concentration was 100 nM, and 5-fold gradient dilution was performed) were added, and the plate was incubated at 4 °C for 30 minutes. A fluorescently labeled goat anti-human IgG secondary antibody (manufacturer: abcam, catalog No. 98596) was then added to detect antibodies bound to the cell surfaces. Antibody-antigen binding dose response curves were generated using geometric values, and four-parameter raw data were plotted using the Graphpad Prism V6.0 software. The EC50 values for the binding of the antibodies to the antigen were determined and are shown in Table 10.

**Table 10**

| Sample No. | EC50 (nM) |
|---|---|
| Antibody 1hu | 0.051 |
| Antibody 1 | 0.041 |
| Antibody 2hu | 0.18 |
| Antibody 2 | 0.14 |
| Antibody 3hu | 0.025 |
| Antibody 3 | 0.025 |
| Antibody 4hu1 | 0.18 |
| Antibody 4hu2 | 0.29 |
| Antibody 4 | 0.16 |
| Positive control | 0.21 |
| hIgG1 | No binding |

The experimental results show that the humanized antibodies exhibited good binding activity to the antigen.

### 2. FACS assay for blocking activity of antibodies against PD-1/PD-L1

The blocking of PD-L1 binding to its ligand PD-1 by the humanized antibodies was measured using a method based on competitive flow cytometry. 293T-hPD1 cells were added to a 96-well plate at 1 × 10⁵ cells/well. Different concentrations of the antibodies to be tested (the initial working concentration was 200 nM, and 4-fold dilution was performed) were mixed with a PD-L1-Biotin dilution (2 µg/mL, 50 µL/well), and the mixtures were incubated at room temperature for 30 minutes. Then the mixtures were added to the cells at 100 µL/well and well mixed. The plate was incubated at 4 °C for 60 minutes, and the blocking activity of the antibodies was measured using a PE fluorescently labeled streptavidin flow cytometry antibody. The mean fluorescence values were measured. Then antibody-antigen blocking reaction curves were generated using geometric values, and four-parameter plots were generated using the Graphpad Prism V6.0 software. The IC50 values were determined and are shown in Table 11 below.

**Table 11**

| Sample No. | IC50 (nM) |
|---|---|
| Antibody 1hu | 4.0 |
| Antibody 1 | 4.2 |
| Antibody 2hu | 3.4 |
| Antibody 2 | 2.7 |
| Antibody 3hu | 1.0 |
| Antibody 3 | 1.1 |
| Antibody 4hu1 | 2.6 |
| Antibody 4hu2 | 12 |
| Antibody 4 | 2.8 |
| Positive control | 2.4 |
| hIgG1 | No blocking |

The experimental results show that the humanized antibodies can block the binding activity of PD-1/PD-L1.

### 3. FACS assay for blocking activity of humanized antibodies against CD-80/PD-L1

The blocking of PD-L1 binding to CD-80 by the antibodies was measured using a competitive flow cytometry method. 293T-PD-L1 cells were added to a 96-well plate at 1 × 10⁵ cells/well. Antibody dilutions with different concentrations (the initial working concentration was 200 nM, and 4-fold dilution was performed) were added to the cells, and the plate was incubated at 4 °C for 30 minutes. After the incubation, the plate was washed with PBS containing 2% FBS and centrifuged at 1200 rpm for 4 minutes, and the supernatants were discarded. Then human-CD80-mFc (2 µg/mL, manufacturer: ACRO) was added at 100 µL/well, and the plate was incubated in the dark at 4 °C for 30 minutes. After the incubation, the plate was washed with PBS containing 2% FBS. A fluorescently labeled goat anti-mouse IgG secondary antibody diluted at 1:200 was added at 100 µL/well, and the plate was incubated in the dark at 4 °C for 30 minutes. After the incubation, the plate was washed with PBS containing 2% FBS and tested on a flow cytometer. The mean fluorescence values were measured.

**Table 12**

| Sample No. | IC50 (nM) |
|---|---|
| Antibody 1hu | 1.8 |
| Antibody 1 | 2.6 |
| Antibody 2hu | 1.7 |
| Antibody 2 | 1.5 |
| Antibody 3hu | 3.2 |
| Antibody 3 | 5.2 |
| Antibody 4hu1 | 1.5 |
| Antibody 4 | 1.5 |
| hIgG1 | No blocking |
| Positive control | 3.0 |

It can be seen from the results that the humanized antibodies can well block the binding of the antigen to CD-80.

### Example 7

A PD-L1 humanized transgenic mouse model of hPD-L1-C57BL/6 female mice (manufacturer: Jiangsu GemPharmatech Co., Ltd., capable of normally expressing humanized PD-L1) was prepared. Cells of the humanized PD-L1 tumor cell line MC-38-hPD-L1 KL3# (manufacturer: Kyinno Biotechnology (Beijing) Co., Ltd.) were inoculated subcutaneously into the right scapula sites of the mice. The inoculation volume was 0.1 mL/mouse, and the number of cells inoculated was 1 × 10⁶ cells/mouse. When the tumor volume reached 75-100 mm³, the mice were divided into groups of 6 and given different drug treatments. The groups were an antibody 1 experimental group, an antibody 2 experimental group, an antibody 3 experimental group, an antibody 4 experimental group, an antibody 5 experimental group, and a vehicle group (PBS). Accordingly, each treatment group of mice was administered a different drug. The dose of the administration was 3 mg/kg; the volume of the administration was 5 µL/g; the route of the administration was intraperitoneal (i.p.) injection; and the frequency of the administration was twice weekly (BIW). Changes in the tumors and body weight of the mice were recorded.

The results in FIG. 6 show the effects of the antibody 1 experimental group through the antibody 5 experimental group and the vehicle group on mouse tumor volume. The experimental results show that: after treatment with different antibody drugs, the tumor volume of each experimental mouse was significantly inhibited; each antibody drug exhibited a good tumor-inhibiting effect.

In the description of the present invention, unless otherwise clearly and specifically defined, "a plurality of" means at least two, e.g., two, three, etc.

In the specification, the reference term "an embodiment", "some embodiments", "an example", "a specific example", or "some examples" means that a specific feature, structure, material, or characteristic described in connection with the embodiment or example is included in at least one embodiment or example of the present invention. In the specification, exemplary descriptions of the foregoing terms are not necessarily intended for the same embodiment or example. Moreover, the specific features, structures, materials, or characteristics described may be combined in any one or more embodiments or examples in a suitable manner. In addition, one skilled in the art may integrate or combine different embodiments or examples described in the specification and features of the different embodiments or examples so long as they are not contradictory to each other.

Although the embodiments of the present disclosure have been shown and described above, it can be understood that the foregoing embodiments are exemplary and should not be understood as limitations on the present invention. A person of ordinary skill in the art can make changes, modifications, replacements, and variations to the foregoing embodiments within the scope of the present invention.

## Claims

1. An anti-PD-L1 nanobody, comprising:
(a) a CDR1, the CDR1 comprising a sequence selected from the group consisting of SEQ ID NOs: 1, 5, and 9, or sequences that have one or two amino acid substitutions, deletions, or additions compared to SEQ ID NOs: 1, 5, and 9;
(b) a CDR2, the CDR2 comprising a sequence selected from the group consisting of SEQ ID NOs: 2, 6, and 10, or sequences that have one or two amino acid substitutions, deletions, or additions compared to SEQ ID NOs: 2, 6, and 10; and
(c) a CDR3, the CDR3 comprising a sequence selected from the group consisting of SEQ ID NOs: 3, 7, 11, and 14, or sequences that have one, two, or three amino acid substitutions, deletions, or additions compared to SEQ ID NOs: 3, 7, 11, and 14.

2. The anti-PD-L1 nanobody according to claim 1, comprising:
(1) a CDR1, which is the amino acid sequence set forth in SEQ ID NO: 1; a CDR2, which is the amino acid sequence set forth in SEQ ID NO: 2; and a CDR3, which is the amino acid sequence set forth in SEQ ID NO: 3; or
(2) a CDR1, which is the amino acid sequence set forth in SEQ ID NO: 5; a CDR2, which is the amino acid sequence set forth in SEQ ID NO: 6; and a CDR3, which is the amino acid sequence set forth in SEQ ID NO: 7; or
(3) a CDR1, which is the amino acid sequence set forth in SEQ ID NO: 9; a CDR2, which is the amino acid sequence set forth in SEQ ID NO: 10; and a CDR3, which is the amino acid sequence set forth in SEQ ID NO: 11; or
(4) a CDR1, which is the amino acid sequence set forth in SEQ ID NO: 1; a CDR2, which is the amino acid sequence set forth in SEQ ID NO: 2; and a CDR3, which is the amino acid sequence set forth in SEQ ID NO: 14.

3. The anti-PD-L1 nanobody according to claim 1 or 2, further comprising framework regions (FRs), wherein the framework regions (FRs) include an FR1, an FR2, an FR3, and an FR4; the FR1, the FR2, the FR3, and the FR4 comprise the following amino acid sequences:
(1) the FR1 comprises a sequence selected from SEQ ID NO: 16, SEQ ID NO: 20, SEQ ID NO: 23, or SEQ ID NO: 26, or a sequence that has one, two, or three conservative amino acid substitutions compared to SEQ ID NO: 16, 20, 23, or 26;
(2) the FR2 comprises a sequence selected from SEQ ID NO: 17, SEQ ID NO: 21, or SEQ ID NO: 24, or a sequence that has one, two, or three conservative amino acid substitutions compared to SEQ ID NO: 17, 21, or 24;
(3) the FR3 comprises a sequence selected from SEQ ID NO: 18, SEQ ID NO: 22, SEQ ID NO: 25, or SEQ ID NO: 27, or a sequence that has one, two, three, or four conservative amino acid substitutions compared to SEQ ID NO: 18, 22, 25, or 27;
(4) the FR4 is selected from SEQ ID NO: 19 or a sequence that has one or two conservative amino acid substitutions compared to SEQ ID NO: 19.

4. The anti-PD-L1 nanobody according to claim 1 or 2, further comprising framework regions (FRs), wherein the framework regions (FRs) include:
(1) an FR1, which is the amino acid sequence set forth in SEQ ID NO: 16; an FR2, which is the amino acid sequence set forth in SEQ ID NO: 17; an FR3, which is the amino acid sequence set forth in SEQ ID NO: 18; and an FR4, which is the amino acid sequence set forth in SEQ ID NO: 19; or
(2) an FR1, which is the amino acid sequence set forth in SEQ ID NO: 20; an FR2, which is the amino acid sequence set forth in SEQ ID NO: 21; an FR3, which is the amino acid sequence set forth in SEQ ID NO: 22; and an FR4, which is the amino acid sequence set forth in SEQ ID NO: 19; or
(3) an FR1, which is the amino acid sequence set forth in SEQ ID NO: 23; an FR2, which is the amino acid sequence set forth in SEQ ID NO: 24; an FR3, which is the amino acid sequence set forth in SEQ ID NO: 25; and an FR4, which is the amino acid sequence set forth in SEQ ID NO: 19; or
(4) an FR1, which is the amino acid sequence set forth in SEQ ID NO: 26; an FR2, which is the amino acid sequence set forth in SEQ ID NO: 24; an FR3, which is the amino acid sequence set forth in SEQ ID NO: 25; and an FR4, which is the amino acid sequence set forth in SEQ ID NO: 19; or
(5) an FR1, which is the amino acid sequence set forth in SEQ ID NO: 16; an FR2, which is the amino acid sequence set forth in SEQ ID NO: 17; an FR3, which is the amino acid sequence set forth in SEQ ID NO: 27; and an FR4, which is the amino acid sequence set forth in SEQ ID NO: 19.

5. The anti-PD-L1 nanobody according to claim 1 or 2, wherein the anti-PD-L1 nanobody comprises:
(a) the amino acid sequence set forth in SEQ ID NO: 4, or SEQ ID NO: 8, or SEQ ID NO: 12, or SEQ ID NO: 13, or SEQ ID NO: 15; or
(b) an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology compared to (a).

6. The anti-PD-L1 nanobody according to claim 1 or 2, wherein the anti-PD-L1 nanobody has the amino acid sequence set forth in SEQ ID NO: 28, or SEQ ID NO: 29, or SEQ ID NO: 30, or SEQ ID NO: 31, or SEQ ID NO: 32.

7. An anti-PD-L1 nanobody, selected from:
i) an antibody that has the amino acid sequence set forth in SEQ ID NO: 4, or SEQ ID NO: 8, or SEQ ID NO: 12, or SEQ ID NO: 13, or SEQ ID NO: 15, or SEQ ID NO: 28, or SEQ ID NO: 29, or SEQ ID NO: 30, or SEQ ID NO: 31, or SEQ ID NO: 32; or
ii) an antibody that has an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology compared to i).

8. A fusion protein, wherein the fusion protein comprises:
a first domain, which is the anti-PD-L1 nanobody according to any of claims 1-7; and
a second domain, which is a functional protein different from the anti-PD-L1 nanobody.

9. The fusion protein according to claim 8, wherein the second domain comprises at least one selected from the following:
1) an immunoglobulin Fc region, preferably a human immunoglobulin Fc region;
2) a cytokine regulatory protein; and
3) an antigen-targeting protein different from PD-L1.

10. An antibody conjugate, comprising:
the anti-PD-L1 nanobody according to any of claims 1-7 or the fusion protein according to claim 8 or 9; and a functional small molecule linked to the anti-PD-L1 nanobody or the fusion protein.

11. An isolated polynucleotide, wherein the isolated polynucleotide encodes the anti-PD-L1 nanobody according to any of claims 1-7 or the fusion protein according to claim 8 or 9.

12. A construct, wherein the construct comprises the isolated polynucleotide according to claim 11.

13. A host cell, wherein the host cell comprises the isolated polynucleotide according to claim 11 or the construct according to claim 12.

14. A method for producing a nanobody or a fusion protein, comprising:
culturing a host cell, wherein the host cell is the host cell according to claim 13; and collecting the nanobody or the fusion protein from the culture.

15. A pharmaceutical composition, comprising:
the anti-PD-L1 nanobody according to any of claims 1-7, or the fusion protein according to claim 8 or 9, or the antibody conjugate according to claim 10; and
a pharmaceutically acceptable carrier.

16. A kit, comprising the anti-PD-L1 nanobody according to any of claims 1-7, or the fusion protein according to claim 8 or 9, or the antibody conjugate according to claim 10.

17. A method for preventing and/or treating disease, comprising:
administering to a subject a prophylactically and/or therapeutically effective amount of the anti-PD-L1 nanobody according to any of claims 1-7, or the fusion protein according to claim 8 or 9, or the antibody conjugate according to claim 10.

18. Use of the anti-PD-L1 nanobody according to any of claims 1-7 or the fusion protein according to claim 8 or 9 or the antibody conjugate according to claim 10 in the preparation of a medicament or a kit.
